# EUROPEAN PATENT APPLICATION

(11) **EP 4 613 708 A1**
(43) Date of publication of application: **10.09.2025**
(21) Application number: 23902038.1
(22) Date of filing: 07.06.2023
(51) Int. Cl.: C01B 39/14, C01B 39/02, B01J 29/80, B01J 37/10, C10G 67/06

(54) **MOLECULAR SIEVE COMPOSITION, AND PREPARATION METHOD THEREFOR AND USE THEREOF**

(30) Priority: 13.12.2022 CN 202211593869
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: QUAN, Hui, Dalian, Liaoning 116045 (CN); WANG, Xiaosi, Dalian, Liaoning 116045 (CN); SONG, Zhaoyang, Dalian, Liaoning 116045 (CN); ZHAO, Wei, Dalian, Liaoning 116045 (CN); ZHANG, Zhiyin, Dalian, Liaoning 116045 (CN); SUN, Guoquan, Dalian, Liaoning 116045 (CN); LIU, Lindong, Dalian, Liaoning 116045 (CN); HU, Shaojian, Dalian, Liaoning 116045 (CN)
(74) Representative: karo IP
(86) International application number: PCT/CN2023/098777
(87) International publication number: WO 2024/124826

(57) **Abstract**

The present application relates to a molecular sieve composition, its preparation and application thereof. The molecular sieve composition comprises a molecular sieve having an embedded structure composed of a molecular sieve having a TON structure and a 5A-type molecular sieve. The molecular sieve composition has dual functions of adsorption and hydroisomerization.

## Description

### Technical Field

The present application belongs to the technical field of catalytic materials, and relates to a molecular sieve composition, its preparation and application thereof.

### Background Art

With the stricter environmental regulations and the rapid development of the mechanical industry, higher and higher requirements are put on the properties of lubricant base oils. The traditional production of lubricant base oil adopts a solvent refining process, and the main two steps of the process are solvent refining to remove non-ideal components such as aromatic hydrocarbon and the like and solvent dewaxing to ensure the low-temperature flow property of the base oil. Meanwhile, further finishing by clay or hydrogenation is normally needed.

At present, the overall crude oil in the world has a deterioration development trend, so that the quantity of the crude oil suitable for producing lubricating oil is gradually reduced, and the development and application of the traditional process are limited due to high energy consumption, serious pollution and other factors. In recent years, the technology for producing lubricating oil by hydrogenation has been developed rapidly. The hydrogenation process is a method for producing lubricant base oils by adopting a hydrocracking process or a hydrotreatinghydroisomerization -hydrofinishing combined process, and has the advantages of high flexibility of raw materials, high yield of the base oil, high economic value of byproducts and the like.

A technical problem of the current hydroisomerization processes is that when a full or wide cut feed is used, it is difficult to simultaneously meet the pour point and viscosity index requirements for both the light and heavy lubricant oil components. In general, to make the pour point of the heavy lubricant component meet to standard, the light lubricant component will be over hydroisomerized, resulting in a loss of viscosity index of the light lubricant component, making it difficult to produce API III light base oil products with a viscosity index above 120.

In the prior art, in order to obtain a high-viscosity-index lubricant base oil product, the objective of producing light lubricant base oil with a high viscosity index is achieved by splitting a raw material into narrow components and then respectively using the narrow components as feedstocks for hydroisomerization in some cases. In some other cases, lubricant base oils with a high viscosity index are produced by a step-wise hydroisomerization -product separation process using whole or wide-cut feedstocks.

US 7,198,710 discloses a method for producing a high-viscosity-index lubricant base oil from fischer-tropsch wax, wherein the fischer-tropsch wax is fractionated to obtain a light component and a heavy component, which are then subjected to hydroisomerization, respectively, to reduce the pour point of the feedstock to obtain a light lubricant base oil having a pour point meeting the requirements, wherein the hydroisomerization heavy component is not meted requirement of pour point, and a solvent dewaxing process is used to further reduce the pour point of the heavy component, and finally a heavy lubricant base oil product having a pour point meeting the requirements is obtained.

CN 102911726A discloses a method for producing a high-viscosity-index lubricant base oil, wherein a wax-containing oil with no need of prefractionation is used as the feedstock for hydroisomerization, and is first passed into a first hydroisomerization reaction zone to conduct a hydroisomerization reaction with an appropriate depth, and the reaction product is fractionated to obtain a light lubricant base oil product with a pour point meeting the requirements and a high viscosity index and a heavy base oil component with a higher pour point. The heavy base oil component is further passed into a second hydroisomerization reaction zone, and the reaction product is fractionated to obtain a heavy lubricant base oil product with a pour point meeting the requirements and a high viscosity index.

### Summary of the Invention

The inventors of the present application found in the course of their research that long-chain isoparaffins and long-side-chain monocyclic naphthenes are ideal components constituting a high-viscosity-index lubricant base oil. In addition to synthetic products such as Fischer-Tropsch synthetic oil (F-T oil) and polyester, raw materials for producing lubricant base oils in natural petroleum products, such as hydrocracking UCO(unconverted oil), hydrotreated wax oil, hydrotreated foots oil and hydrotreated slack wax, and other waxy raw materials, generally contain long-chain isoparaffins, long-side-chain monocyclic naphthenes and long-chain monocyclic aromatics, and also contain a certain amount of non-ideal components, such as naphthenes and aromatics with two or more rings, and such non-ideal components cannot be converted into high viscosity index components in the pour point depressing process through hydroisomerization, and the improvement of the viscosity index of the hydroisomerization reaction product, namely the lubricant base oil, is severely limited due to the existence of the non-ideal components. The inventors propose that if the non-ideal components in the raw material can be removed in advance without subsequent an hydroisomerization reaction treatment, on one hand, the viscosity index of the hydroisomerization reaction product, namely the lubricant base oil, can be improved, and on the other hand, the hydrogen consumption of the hydroisomerization reaction process can be greatly reduced, so that the operation cost of the plant and the production cost can be reduced. The present application is completed based on the above findings.

To solve the problems and overcome the defects in the prior art, a major object of the present application is to provide a method and a system for producing lubricant base oil, wherein non-ideal components with a low viscosity index such as naphthenes with two or more rings and a part of aromatics in the lubricant base oil raw material are separated through a selective adsorption process, so that only the pre-treated raw material rich in long-chain isoparaffins, long-side-chain monocyclic naphthenes and long-chain monocyclic aromatics is subjected to an isomerization pour point depression reaction to obtain a high-viscosity-index lubricant base oil product.

To achieve the above object, in an aspect, the present application provides a molecular sieve composition comprising a molecular sieve having an embedded structure composed of a molecular sieve having a TON structure and a 5A-type molecular sieve.

### Technical effects

Compared with the prior art, the present application has the following advantages:
1. The molecular sieve composition according to the present application is formed by combining a 5A-type molecular sieve and a molecular sieve having a TON structure in a mutually mosaic manner, wherein the 5A-type molecular sieve has a good adsorption function and can enrich alkane, the molecular sieve having a TON structure has a specific pore structure and an appropriate acidity and is beneficial to selective isomerization of alkanes, and the molecular sieve composition according to the present application has all of the above special properties at the same time, and still shows good adsorption performance after loading active metal. After the catalyst prepared by taking the molecular sieve composition according to the present application as a component is subjected to isomerization reaction and regeneration, the molecular sieve still shows good stability.
2. In the method for producing a lubricant base oil according to the present application, the raw material is first subjected to an adsorption treatment to separate non-ideal components, so as to ensure that only a feedstock rich in long-chain isoparaffins, long-side-chain monocyclic naphthenes, long-chain monocyclic aromatics and other ideal components is subjected to an hydroisomerization pour point reducing reaction, so that a high-viscosity-index lubricant base oil product is obtained, and the participation of the non-ideal components in hydrogenation reaction is greatly reduced, that is the load of an hydroisomerization reaction process is reduced, the hydrogen consumption of the process is lowered, and the economy of the plant is improved. In addition, in the research process, the applicant found that the addition of 5A-type molecular sieve in currently used hydroisomerization catalyst provides a selective adsorption effect on the raw material of the lubricant base oil in the absence of hydrogen, which can separate out the non-ideal components with a low viscosity index such as naphthenes with two or more rings and a part of aromatics in the raw material, reduce the content of low-viscosity-index components in the lubricant base oil product from the aspect of the raw material, and improve the viscosity index of the lubricant base oil.
3. In the method for producing a lubricant base oil according to the present application, the molecular sieve composition has the selective adsorption capacity on long-chain isoparaffins, long-side-chain monocyclic naphthenes and long-chain monocyclic aromatics and the selective isomerization capacity on alkane in the presence of hydrogen under appropriate reaction conditions at the same time, so that the production process of the high-viscosity-index lubricant base oil is simplified, the plant investment is reduced, and the hydrogen consumption of the process is reduced.
4. In the method for producing a lubricant base oil according to the present application, the adsorption separation efficiency is expressed by the change of the refractive index of the raw material before and after the adsorption treatment, which parameter is simple and convenient to measure, and the effect of the separation of ideal components such as long-chain isoparaffins, long-side-chain monocyclic naphthenes, long-chain monocyclic aromatics and the like in the raw material can be intuitively reflected thereby.
5. Compared with a mechanical mixture of the molecular sieve having a TON structure and the 5A-type molecular sieve, the mutually mosaic combination formed by the molecular sieves in the molecular sieve having an embedded structure is more tight, so that the diffusion distance of n-paraffins from the 5A-type molecular sieve having an adsorption function to the molecular sieve having a TON structure having an isomerization function can be greatly shortened, and a significant adsorptioncatalysis synergistic effect can be obtained.

### Brief Description of the Drawings

Fig. 1 is a schematic flow chart of a method for producing a lubricant base oil according to a first embodiment of the present application.
Fig. 2 is a schematic flow chart of a method for producing a lubricant base oil according to a second embodiment of the present application.

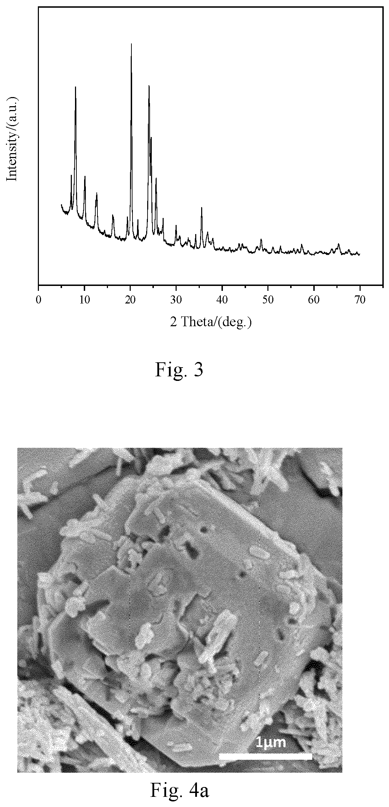

Fig. 3 is an XRD pattern of the molecular sieve having an embedded structure obtained in Example 2.

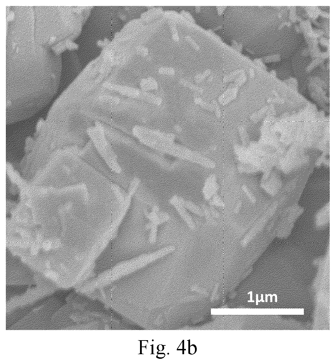

Figs. 4a and 4b are SEM images of the molecular sieves having an embedded structure obtained in Example 2 and Example 3, respectively. In these drawings, the cubes are 5A-type molecular sieves, while the short rod shaped crystals are molecular sieves having a TON structure. As can be seen from these drawings, the molecular sieve having a TON structure present in the form of short rods is embedded onto the surface of the 5A-type molecular sieve cubes by covering at least a part of the surface of the 5A-type molecular sieve cubes.

### Detailed Description of the Invention

The present application will be illustrated in detail hereinbelow with reference to embodiments thereof, but it should be noted that the scope of the present application is not limited by those embodiments, but is defined by the appended claims.

All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

Where a material, substance, method, step, device, component, or the like is described herein as "commonly known to those skilled in the art", "prior art" or the like, it is to be understood that said material, substance, method, step, device and component cover not only those conventionally used in the art at the time of filing the present application, but also those not commonly used at present but will become commonly known in the art to be suitable for a similar purpose.

Throughout the context of the specification and claims, unless explicitly stated otherwise, the term "comprise" or its variations such as "comprises" or "comprising", etc., shall be understood as including the element or component explicitly mentioned, without excluding any other element or component.

In the context of the present application, spatially relative terms, such as "lower," "below," "beneath," "upper," "above," "over," and the like, may be used, for convenience, to describe the relationship between one element or feature and another element or feature in the figures. It can be appreciated that the spatially relative terms are intended to cover different orientations of the article in use or operation in addition to that shown in the figures. For example, if the article shown in the figures is turned over, an element described as "below" or "beneath" another element or feature would then be oriented "above" the element or feature. Thus, the exemplary term "below" may cover both the orientation of "below" and the orientation of "above". The article may be otherwise oriented (e.g., rotated 90 degrees or at other orientations) and the spatially relative terms used herein should be interpreted accordingly.

In the context of the present application, the terms "first", "second", and the like are used to distinguish two different elements or parts, while are not used to define a particular positional or relative relationship. In other words, in some examples, the terms "first" and "second" and the like may be used interchangeably.

In the context of the present application, all numerical values of a parameter (e.g., quantity or condition) shall be understood as being modified in all instances by the term "about", no matter whether or not the term "about" is actually present before the numerical value.

In the context of the present application, unless explicitly stated otherwise, both the silica-alumina material and the catalyst have been subjected to a calcination treatment, sometimes referred to as "calcined form", prior to the measurement. Here, the conditions of the calcination treatment include: air atmosphere, a calcining temperature of 600 °C, and a calcining time of 3 h or more.

In the context of the present application, long-side-chain hydrocarbon refers particularly to one or more of long-side-chain isoparaffins, long-side-chain monocyclic naphthenes and long-side-chain monocyclic aromatics, excluding other hydrocarbons with long side chains. In addition, according to the present application, the term "long side chain" means a side chain of C8-22 (preferably C10-18) linear alkyl group.

In the context of the present application, a mechanical mixture refers to a mixture of two or more materials obtained by means of mechanical mixing. Here, the mechanical mixing includes simple mixing, grinding, slurrying, and the like.

In the context of the present application, a molecular sieve having an embedded structure refers to a composite crystal of one or more molecular sieve crystals with another one or more molecular sieves embedded onto the surface thereof or inside the crystals, which has two or more molecular sieve structural characteristics. Compared with mechanical mixtures, different molecular sieves are combined more tightly in the mosaic structure, and an integrated composite structure at a molecular level is really formed.

In the context of the present application, the refractive index is characterized by a Mettler refractometer R5, the long chain hydrocarbon content is characterized by an Agilent gas chromatography 7890, the XRD pattern of the sample is characterized by a D/max-2500 full-automatic rotating target X-ray diffractometer, the specific surface area, pore volume and average pore diameter of the molecular sieve having an embedded structure and the specific surface area, pore volume and average pore diameter of the molecular sieve composition are tested and characterized by an ASAP 2405 physical adsorption apparatus according to the N₂ adsorption-desorption method, and the calcium content of calcium-type molecular sieve is characterized by X-ray fluorescence diffraction.

In the context of the present application, the quantitative analysis of the molecular sieve having a TON structure in the molecular sieve having an embedded structure is determined by XRD.

In the context of the present application, unless specifically stated otherwise, all percentages, parts, ratios, etc. are expressed by weight, all pressures given are gauge pressures, and the weight or content is calculated on a dry basis.

In the context of the present application, any two or more embodiments of the present application may be arbitrarily combined, and the resulting technical solution forms a part of the initial disclosure of the present application and falls within the scope of the present application.

According to an embodiment of the present application, there is provided a molecular sieve composition comprising a molecular sieve having an embedded structure composed of a molecular sieve having a TON structure and a 5A-type molecular sieve. Preferably, the molecular sieve having a TON structure is embedded onto at least a part of the surface of the 5A-type molecular sieve with a predetermined surface coverage. From the viewpoint of achieving the technical effect of the present application, a suitable surface coverage may be 0.5% or more or 1% or more, and 50% or less or 20% or less, but the present application is not limited thereto.

According to an embodiment of the present application, the calcined form of the molecular sieve having an embedded structure shows an XRD pattern substantially as shown in Table I or Table II below.

**Table I**

| **2θ** | **d-spacing (nm)** | **Relative strength** |
|---|---|---|
| 7.184 | 1.204±0.020 | W-S |
| 8.229 | 1.090±0.020 | M-VS |
| 10.133 | 0.872±0.006 | M |
| 12.473 | 0.709±0.008 | W-M |
| 12.685 | 0.694±0.006 | W-M |
| 16.140 | 0.549±0.004 | W |
| 19.338 | 0.459±0.007 | W |
| 20.275 | 0.438±0.008 | M-VS |
| 21.699 | 0.409±0.008 | W-S |
| 24.098 | 0.369±0.005 | VS |
| 24.563 | 0.362±0.004 | M-VS |
| 25.680 | 0.347±0.005 | W-M |
| 26.148 | 0.341±0.003 | W |
| 27.152 | 0.328±0.002 | W |
| 29.997 | 0.298±0.003 | W |
| 34.237 | 0.262±0.005 | W-M |
| 35.537 | 0.252±0.006 | W-M |

**Table II**

| **2θ** | **d-spacing (nm)** | **Relative strength** |
|---|---|---|
| 7.184 | 1.204±0.020 | W-S |
| 8.229 | 1.090±0.020 | M-VS |
| 10.133 | 0.872±0.006 | M |
| 12.473 | 0.709±0.008 | W-M |
| 12.685 | 0.694±0.006 | W-M |
| 14.399 | 0.615±0.006 | W |
| 16.140 | 0.549±0.004 | W |
| 17.630 | 0.503±0.006 | W |
| 19.338 | 0.459±0.007 | W |
| 20.275 | 0.438±0.008 | M-VS |
| 21.699 | 0.409±0.008 | W-S |
| 24.098 | 0.369±0.005 | VS |
| 24.563 | 0.362±0.004 | M-VS |
| 25.680 | 0.347±0.005 | W-M |
| 26.148 | 0.341±0.003 | W |
| 26.675 | 0.334±0.004 | W |
| 27.152 | 0.328±0.002 | W |
| 29.997 | 0.298±0.003 | W |
| 30.725 | 0.290±0.004 | W-M |
| 32.624 | 0.274±0.005 | W |
| 34.237 | 0.262±0.005 | W-M |
| 35.537 | 0.252±0.006 | W-M |
| 36.835 | 0.244±0.004 | W |
| 38.038 | 0.236±0.004 | W |

According to the present application, the intensity value of the strongest diffraction peak in the XRD pattern is set as 100, and W represents weak, namely a relative intensity from more than 0 to less than or equal to 20; M represents medium, namely a relative strength from more than 20 to less than or equal to 40; S represents strong, namely a relative strength from more than 40 to less than or equal to 60; VS represents very strong, that is a relative intensity from more than 60 to less than or equal to 100.

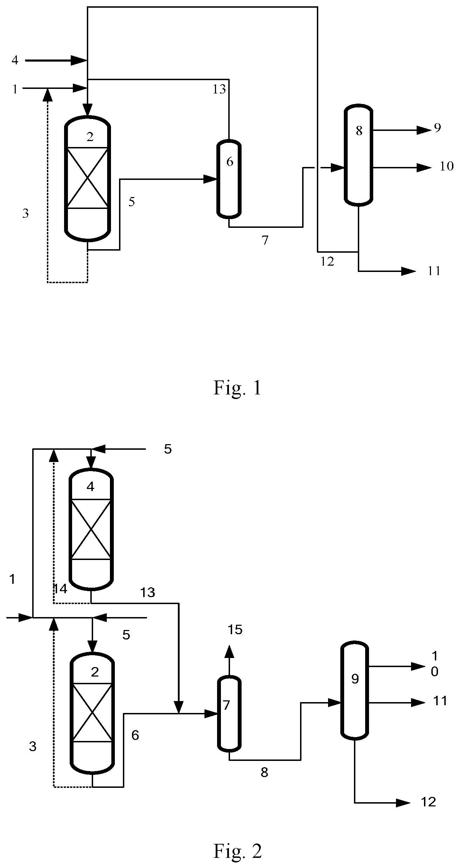

According to an embodiment of the present application, the calcined form of the molecular sieve having an embedded structure has an XRD pattern substantially as shown in figure 1.

According to an embodiment of the present application, the molecular sieve having an embedded structure has a specific surface area of 300 m²/g to 600 m²/g and a pore volume of 0.15 cm³/g to 0.40 cm³/g.

According to an embodiment of the present application, the molecular sieve composition has a specific surface area of 200 m²/g to 550 m²/g and a pore volume of 0.25 cm³/g to 0.60 cm³/g.

According to an embodiment of the present application, in the molecular sieve having an embedded structure, the weight ratio of the 5A-type molecular sieve to the molecular sieve having a TON structure is 1 : 80 to 3 : 1, preferably 1 : 30 to 1 : 1, from the viewpoint of providing a suitable surface coverage.

According to an embodiment of the present application, the content of said molecular sieve having a TON structure is between 10% and 80% by weight, preferably between 20% and 60% by weight, and the content of said 5A-type molecular sieve is between 1% and 50% by weight, preferably between 2% and 20% by weight, relative to the total weight of the molecular sieve composition as 100% by weight.

According to an embodiment of the present application, the content of molecular sieve having an embedded structure is between 10% and 90% by weight, preferably between 20% and 70% by weight, relative to the total weight of the molecular sieve composition as 100% by weight.

According to an embodiment of the present application, the molecular sieve composition further comprises an inorganic refractory oxide and an active metal component.

According to an embodiment of the present application, the active metal component is present in an amount of 0.05wt% to 5.0wt%, preferably 0.1wt% to 1.0wt%, calculated on the basis of metal element, relative to the total weight of the molecular sieve composition as 100 wt%.

According to an embodiment of the present application, the inorganic refractory oxide is one or more selected from the group consisting of alumina, titania, boria, silica, zirconia and magnesia, preferably alumina.

According to an embodiment of the present application, the active metal component is at least one selected from the noble metals of Group VIII of the periodic table, preferably at least one selected from Pt and Pd, particularly Pt.

According to an embodiment of the present application, the molecular sieve having a TON structure is one or more selected from the group consisting of ZSM-22, Theta-1, ISI-1, KZ-2 and NU-10, and preferably ZSM-22.

According to an embodiment of the present application, the 5A-type molecular sieve is selected from 5A molecular sieves.

According to an embodiment of the present application, the molecular sieve composition has dual functions of adsorption and hydroisomerization, and can selectively enrich long-side-chain hydrocarbon, and then perform further hydroisomerization reaction on them, while avoiding excessive isomerization.

According to an embodiment of the present application, there is provided a method for preparing a molecular sieve having an embedded structure, which can be used for preparing the molecular sieve having an embedded structure composed of a molecular sieve having a TON structure and a 5A-type molecular sieve as described hereinabove.

According to an embodiment of the present application, the method for preparing the molecular sieve having an embedded structure comprises the steps of:
(1) contacting a silicon source, an aluminum source, and an alkali source in the presence of a templating agent, a molecular sieve having a TON structure, and water to obtain a gel mixture, and
(2) carrying out hydrothermal crystallization on the gel mixture, and then washing, drying and calcining to obtain a first molecular sieve having an embedded structure.

According to an embodiment of the present application, the method further comprises the steps of:
(3) carrying out calcium exchange on the first molecular sieve having an embedded structure, and then washing, drying and calcining to obtain a second molecular sieve having an embedded structure.

According to an embodiment of the present application, in step (1), the silicon source is at least one selected from the group consisting of water glass, sodium silicate, methyl orthosilicate, ethyl orthosilicate, propyl orthosilicate and butyl orthosilicate.

According to an embodiment of the present application, in step (1), the alkali source is at least one selected from alkali metal hydroxides, preferably sodium hydroxide.

According to an embodiment of the present application, in step (1), the aluminum source is at least one selected from the group consisting of sodium metaaluminate, aluminum isopropoxide, aluminum sulfate, aluminum hydroxide, alumina, and pseudo-boehmite.

According to an embodiment of the present application, in step (1), the templating agent is at least one selected from polyethylene oxide triblock copolymer (P123), dimethyloctadecyl[3-(trimethoxysilyl)propyl] ammonium chloride (TPOAC).

According to an embodiment of the present application, in step (1), the molecular sieve having a TON structure is one or more selected from ZSM-22, Theta-1, ISI-1, KZ-2 and NU-10, and is preferably ZSM-22.

According to an embodiment of the present application, in step (1), the operating conditions of the contacting include: under stirring, a temperature of 15-30 °C and a time period of 1-4 h.

According to an embodiment of the present application, in step (1), the molar ratio of the alkali source (calculated as oxide), the silicon source (calculated as SiO₂), the aluminum source (calculated as Al₂O₃), the templating agent and water is (0.5-2.5) : 1 : (0.4-0.7) : (0.001-0.08) : (30-200), wherein the amount of the molecular sieve having a TON structure is 0.3-8 times of the amount of the silicon source, and preferably 1-7 times.

According to an embodiment of the present application, in step (2), the hydrothermal crystallization temperature is 60-120 °C, and the crystallization time is 2-16 h.

According to an embodiment of the present application, in step (2), the drying temperature is 80-200 °C and the drying time is 2-24 h.

According to an embodiment of the present application, in step (2), the calcining temperature is 400 °C to 600 °C and the calcining time is 2h to 12 h.

According to an embodiment of the present application, in step (3), the operating conditions of the calcium exchange include: an exchange temperature of 60-100 °C, an exchange time of 1-12 h, and a calcium ion concentration in the calcium exchange solution of 0.1-2.5 mol/L.

According to an embodiment of the present application, in step (3), the drying temperature is 80-150 °C and the drying time is 2-12 h.

According to an embodiment of the present application, in step (3), the calcining temperature is 300 °C to 500 °C and the calcining time is 2h to 8 h.

According to an embodiment of the present application, there is also provided a method for producing a lubricant base oil, in which the molecular sieve composition described hereinabove is used.

According to an embodiment of the present application, the method for producing a lubricant base oil comprises the steps of:
(1) contacting a feedstock comprising a long-side-chain hydrocarbon with a molecular sieve composition as described hereinabove, for example in the presence or absence of hydrogen, to obtain an enriched molecular sieve composition and a raffinate having a reduced content of the long-side-chain hydrocarbon,
(2) subjecting the enriched molecular sieve composition to a treatment under hydroisomerization conditions to obtain an isomerization product mixture and a depleted molecular sieve composition, and
(3) separating the isomerization product mixture to obtain the lubricant base oil.

According to an embodiment of the present application, the contacting of the feedstock with the molecular sieve composition is repeated a plurality of times until the refractive index of the raffinate is 0.1% to 5%, preferably 0.5% to 2.5%, higher than the refractive index of the feedstock.

According to an embodiment of the present application, the raffinate can be further processed and can also be used as white oil.

According to an embodiment of the present application, step (1) may be carried out in one or more reaction zones (e.g., in reaction zones including a first reaction zone and a second reaction zone, etc.), and step (2) may be carried out in one or more reaction zones (e.g., in reaction zones including a first reaction zone and a second reaction zone, etc.). Preferably, step (1) and step (2) are carried out in the same reaction zone.

According to an embodiment of the present application, more specifically, the method for producing a lubricant base oil of the first embodiment comprises:
(1) passing the feedstock into a first reaction zone filled with the molecular sieve composition, and recycling a first stream obtained after treatment to the first reaction zone for further treatment;
(2) stopping the feeding of the feedstock and the first stream to the first reaction zone when the refractive index (20 °C) of the first stream is 0.1-5% higher than the refractive index (20 °C) of the feedstock;
(3) introducing hydrogen into the first reaction zone, subjecting the adsorbate to an hydroisomerization reaction under the action of the molecular sieve composition and the hydrogen, and separating the resulted reaction product to obtain a lubricant base oil product.

A method for producing a lubricant base oil according to a second embodiment comprises:
(1) passing the feedstock into a first reaction zone filled with the molecular sieve composition, and recycling a first stream obtained after treatment to the first reaction zone for further treatment;
(2) stopping the feeding of the feedstock and the first stream to the first reaction zone when the refractive index (20 °C) of the first stream is 0.1-5% higher than the refractive index (20 °C) of the feedstock;
(3) introducing hydrogen into the first reaction zone, subjecting the adsorbate to an hydroisomerization reaction under the action of the molecular sieve composition and the hydrogen, and separating the resulted reaction product to obtain a lubricant base oil product;
(4) introducing the feedstock into a second reaction zone filled with the molecular sieve composition, and recycling a second stream obtained after treatment to the second reaction zone for further treatment;
(5) stopping the feeding of the feedstock and the second stream to the second reaction zone when the refractive index (20 °C) of the second stream is 0.1-5% higher than the refractive index (20 °C) of the feedstock;
(6) introducing hydrogen into the second reaction zone, subjecting the adsorbate to an hydroisomerization reaction under the action of the molecular sieve composition and the hydrogen, and separating the resulted reaction product to obtain a lubricant base oil product; and
(7) reintroducing the feedstock into the first reaction zone and repeating the steps (1) to (6).

According to an embodiment of the present application, the first reaction zone and the second reaction zone can be connected in parallel and switched according to the actual need during the production process. The first reaction zone may be provided with one or more reactors, the reactor can be one or more of existing fixed bed hydrogenation reactors, fluidized bed hydrogenation reactors and slurry bed hydrogenation reactors, and preferably fixed bed hydrogenation reactor. The second reaction zone may be provided with one or more reactors, the reactor can be one or more of existing fixed bed hydrogenation reactors, fluidized bed hydrogenation reactors and slurry bed hydrogenation reactors, and preferably fixed bed hydrogenation reactor.

According to an embodiment of the present application, the molecular sieve compositions used in the first and second reaction zones are the same or different, preferably the same.

According to an embodiment of the present application, the feedstock is one or more selected from the group consisting of hydrocracking UCO, hydrotreated wax oil, hydrotreated bright stock, hydrotreated slack wax and hydrotreated foots oil.

According to an embodiment of the present application, in step (1), the molecular sieve composition selectively adsorbs long-side-chain hydrocarbon in the feedstock by the contacting, the long-side-chain hydrocarbon is selectively adsorbed in the pore channels of the molecular sieve composition, and by cyclic adsorption of the feedstock, long-side-chain hydrocarbon is continuously accumulated on the molecular sieve composition, thereby producing a molecular sieve composition adsorbed with long-side-chain hydrocarbon (referred to as enriched molecular sieve composition) and a raffinate. The contacting is carried out in the absence of hydrogen. Herein, the absence of hydrogen means that no hydrogen is actively or intentionally introduced or added into the contacting reaction system.

According to an embodiment of the present application, in step (1), the operating conditions of the contacting include: a temperature of 40-250 °C, preferably 60-200 °C, a pressure of 0.01-0.5 MPa, preferably 0.08-0.1 MPa, and a volume space velocity of 0.05 h⁻¹ to 5.0 h⁻¹ , preferably 0.1 h⁻¹ to 2.0 h⁻¹ .

According to an embodiment of the present application, in step (2), the operating conditions of the hydroisomerization reaction include: the presence of hydrogen, a reaction temperature of 200-420 °C, preferably 270-380 °C, a hydrogen partial pressure of 1.0-20.0 MPa, preferably 3.0-15.0 MPa, a reaction time of 0.1-10 h, preferably 0.3-2 h, and a volume ratio of hydrogen to oil of 100 : 1 to 1500 : 1, preferably 100 : 1 to 400 : 1.

According to an embodiment of the present application, in step (3), the separation comprises the steps of:
(3-1) subjecting the isomerization product mixture to gas-liquid separation to obtain a gas stream and a liquid stream, optionally recycling at least a part of the gas stream to step (1) as at least a part of the feedstock, and
(3-2) subjecting said liquid stream to fractionation to obtain said lubricant base oil, preferably to obtain a light lubricant base oil, a medium lubricant base oil and a heavy lubricant base stock, optionally recycling at least a part of said heavy lubricant base stock to the step (1) as at least a part of said feedstock.

According to an embodiment of the present application, in step (3-1), the gas-liquid separation is performed by vacuum distillation under conditions including: a pressure at the top of the distillation column of 1-100 mm Hg, preferably 2-20 mm Hg, and a temperature at the bottom of the distillation column of 200-370 °C, preferably 290-350 °C.

According to an embodiment of the present application, in step (3-2), the fractionation is performed by vacuum distillation under conditions including: a pressure at the top of the distillation column of 1-100 mm Hg, preferably 2-20 mm Hg, and a temperature at the bottom of the distillation column of 200-370 °C, preferably 290-350 °C.

According to an embodiment of the present application, there is also provided a system for producing a lubricant base oil, including:
one or more reaction zones (e.g., including a first reaction zone and a second reaction zone, etc.) packed with a molecular sieve composition as described hereinabove and configured to contact a feedstock comprising a long-side-chain hydrocarbon with the molecular sieve composition as described hereinabove to obtain an enriched molecular sieve composition and a raffinate having a reduced content of the long-side-chain hydrocarbon, and then treating the enriched molecular sieve composition under hydroisomerization conditions to obtain a hydroisomerization product mixture and a depleted molecular sieve composition,
a gas-liquid separation zone configured to carry out a gas-liquid separation on the isomerization product mixture to obtain a gas stream and a liquid stream, and
a fractionation column configured to fractionate the liquid stream to obtain the lubricant base oil.

According to an embodiment of the present application, more specifically, the system for producing a lubricant base oil comprises:
a first reaction zone for receiving the feedstock, wherein the first reaction zone is filled with the molecular sieve composition, the feedstock is subjected to adsorption reaction in the presence of the molecular sieve composition, a first stream obtained after adsorption treatment is recycled to the first reaction zone for further treatment, when the refractive index (20 °C) of the first stream is 0.1-5% higher than the refractive index (20 °C) of the feedstock, the feeding of the feedstock and the first stream to the first reaction zone are stopped, hydrogen is introduced into the first reaction zone at the same time, the adsorbate is subjected to a hydroisomerization reaction under the action of the molecular sieve composition and the hydrogen, and the reaction product is separated to obtain a lubricant base oil product;
a second reaction zone for receiving the feedstock when the feeding to the first reaction zone is stopped, wherein the second reaction zone is filled with the molecular sieve composition, the feedstock is subjected to adsorption reaction in the presence of the molecular sieve composition, a second stream obtained after the adsorption treatment is recycled to the second reaction zone for further treatment, when the refractive index (20 °C) of the second stream is 0.1-5% higher than the refractive index (20 °C) of the feedstock, the feeding of the feedstock and the second stream to the second reaction zone is stopped, the feeding of the feedstock is switched back to the first reaction zone, hydrogen is introduced into the second reaction zone at the same time, the adsorbate is subjected to a hydroisomerization reaction under the action of the molecular sieve composition and the hydrogen, and the reaction product is separated to obtain a lubricant base oil product;
a gas-liquid separation zone for receiving the hydroisomerization reaction product from the first separation zone or the second separation zone, where a gas stream and a liquid stream are obtained after separation; and
a fractionation column for receiving the liquid stream from the gas-liquid separation zone, where a light lubricant base oil, a medium lubricant base oil and a heavy lubricant base stock are obtained after fractionation.

The first reaction zone and the second reaction zone can be connected in parallel and switched according to the actual need during production process. The first reaction zone may be provided with one or more reactors, the reactor can be one or more of existing fixed bed hydrogenation reactors, fluidized bed hydrogenation reactors and slurry bed hydrogenation reactors, and preferably fixed bed hydrogenation reactor. The second reaction zone may be provided with one or more reactors, the reactor can be one or more of existing fixed bed hydrogenation reactors, fluidized bed hydrogenation reactors and slurry bed hydrogenation reactors, and preferably fixed bed hydrogenation reactor.

The gas-liquid separation zone can be any device existing in the field that can realize the separation of a gas phase and a liquid phase, such as a gas-liquid separator, a flash tower and the like.

The present application is illustrated in further detail below with reference to the drawings, but the present application is not limited thereto.

As shown in Fig. 1, in the method for producing a lubricant base oil according to the first embodiment of the present application, the feedstock 1 is fed into the first reaction zone 2 (at this moment an adsorption reaction occurs in the first reaction zone), the feedstock 1 is reacted under the action of the molecular sieve composition to obtain a first stream 3, the first stream 3 is recycled to the first reaction zone 2, when the refractive index of the first stream 3 is 0.1-5% higher than the refractive index of the feedstock 1, the feeding of the feedstock 1 and the first stream 3 to the first reaction zone 2 are stopped, at the same time hydrogen 4 is introduced into the first reaction zone 2, so that the adsorbate undergoes a hydroisomerization reaction under the action of the molecular sieve composition and the hydrogen 4, the resulted reaction product 5 is passed to a gas-liquid separation zone 6 for separation to obtain a gas stream 13 and a liquid stream 7, and the gas stream 13 is recycled to the first reaction zone 2 (at this moment a hydroisomerization reaction occurs in the first reaction zone); the liquid stream 7 is further passed to a fractionation column 8 for separation to obtain a light lubricant base oil 9, a medium lubricant base oil 10 and a heavy lubricant base stock, the heavy lubricant base stock is divided into two streams, wherein one stream of heavy lubricant base stock 11 is discharged from the system, and the other stream of heavy lubricant base stock 12 can be recycled to the first reaction zone 2 for further reaction.

As shown in Fig. 2, in the method for producing a lubricant base oil according to the second embodiment of the present application, the feedstock 1 is fed into the first reaction zone 2 (at this moment an adsorption reaction occurs in the first reaction zone), the feedstock 1 is reacted under the action of the molecular sieve composition to obtain a first stream 3, the first stream 3 is recycled to the first reaction zone 2, when the refractive index of the first stream 3 is 0.1-5% higher than the refractive index of the feedstock 1, the feeding of the feedstock 1 and the first stream 3 to the first reaction zone 2 are stopped, at the same time hydrogen 5 is introduced into the first reaction zone 2, so that the adsorbate undergoes a hydroisomerization reaction under the action of the molecular sieve composition and the hydrogen 5 to obtain a reaction product 6, the reaction product 6 is passed into a gas-liquid separation zone 7 for gas-liquid separation to obtain a gas stream 15 and a liquid stream 8, and the gas stream 15 is recycled to the first reaction zone 2 (at this moment a hydroisomerization reaction occurs in the first reaction zone); the liquid stream 8 is further passed to a fractionation column 9 for separation to obtain a light lubricant base oil 10, a medium lubricant base oil 11 and a heavy lubricant base stock 12, and a part or all of the heavy lubricant base stock 12 can be recycled to the first reaction zone 2 for further reaction. At the same time, the first reaction zone 2 is switched to a hydroisomerization reaction mode, while the feeding of the feedstock 1 is switched to a second reaction zone 4, the feedstock 1 is reacted under the action of the molecular sieve composition to obtain a second stream 14, the second stream 14 is recycled to the second reaction zone 4, when the refractive index of the second stream 14 is 0.1-5% higher than the refractive index of the feedstock 1, the feeding of the feedstock 1 and the second stream 14 to the second reaction zone 4 are stopped, at the same time hydrogen 5 is introduced into the second reaction zone 4, so that the adsorbate undergoes a hydroisomerization reaction under the action of the molecular sieve composition and the hydrogen 5 to obtain a reaction product 13, the reaction product 13 is also passed to the gas-liquid separation zone 7 for gas-liquid separation to obtain the gas stream 15 and the liquid stream 8, and the gas stream 15 is recycled to the first reaction zone 2 (at this moment a hydroisomerization reaction occurs in the first reaction zone); the liquid stream 8 is further passed to the fractionation column 9 for separation to obtain the light lubricant base oil 10, the medium lubricant base oil 11 and the heavy lubricant base stock 12, and a part or all of the heavy lubricant base stock 12 can be recycled to the second reaction zone 4 for further reaction.

### Examples

The present application will be described in further detail with reference to examples, but the present application is not limited to these examples.

In the following examples and comparative examples, all the reagents and raw materials are either commercially available or can be prepared according to the knowledge already known in the art.

### Example 1

48 g of sodium hydroxide, 208 g of ethyl orthosilicate, 73.8 g of sodium metaaluminate, 3.97 g of dimethyloctadecyl [3-(trimethoxysilyl)propyl] ammonium chloride (TPOAC) and 720 g of water were uniformly mixed and charged into a crystallization kettle, 36 g of ZSM-22 molecular sieve was added into the crystallization kettle and stirred, the reaction kettle was sealed, and the reaction mixture was heated to 120 °C for hydrothermal crystallization for 3 hours. After the crystallization was completed, the crystallization product obtained was washed, dried at 110 °C for 5 hours, and calcined at 500 °C for 4 hours to obtain a first molecular sieve Z1 having an embedded structure. Then, the molecular sieve Z1 was subjected to ion exchange in 0.8 mol/L CaCl₂ solution at 65 °C for 6 hours, then to suction filtration and washing, dried for 4 hours at 100 °C, and calcined for 3 hours at 450 °C, to obtain a second molecular sieve GZ-1 having an embedded structure, of which the specific surface area was 584 m²/g, the pore volume was 0.22 mL/g, and the mass fraction of the molecular sieve having a TON structure was 85%.

210g of the molecular sieve GZ-1 having an embedded structure obtained, 90 g of pseudo-boehmite (dry basis) and 20 g of sesbania powder were fully and uniformly mixed, 9 ml of concentrated nitric acid (with a mass fraction of 65%) and a proper amount of water were added, and the mixture was fully kneaded and then shaped by extrusion. The shaped carrier was dried at 100 °C for 4h, and calcined at 550 °C for 4h to obtain a carrier ES-1. Then, the carrier was impregnated with the noble metal Pt via saturated impregnation, wherein the Pt load was 0.28 wt% of the carrier, the resultant was dried for 6 h at 100 °C, and calcined for 3 h at 500 °C to obtain a catalyst of the present application, designated as E-1.

### Example 2

128 g of sodium hydroxide, 208 g of ethyl orthosilicate, 82 g of sodium metaaluminate, 14.88 g of dimethyloctadecyl [3-(trimethoxysilyl)propyl] ammonium chloride (TPOAC) and 1800 g of water were uniformly mixed and charged into a crystallization kettle, 300 g of ZSM-22 molecular sieve was added into the crystallization kettle and stirred, the reaction kettle was sealed, and the reaction mixture was heated to 110 °C for hydrothermal crystallization for 6 hours. After the crystallization was completed, the crystallization product obtained was washed, dried at 110 °C for 6 hours, and calcined at 500 °C for 5 hours to obtain a first molecular sieve Z2 having an embedded structure. Then, the molecular sieve Z2 was subjected to ion exchange in 1.8 mol/L CaCl₂ solution at 90 °C for 4 h, then to suction filtration and washing, then dried at 100 °C for 4 h, and calcined at 400 °C for 3h to obtain a second molecular sieve GZ-2 having an embedded structure, of which the specific surface area was 452 m²/g, the pore volume was 0.25 mL/g, and the mass fraction of the molecular sieve having a TON structure was 85%.

A catalyst E-2 of the present application was prepared as described in Example 1, except that the molecular sieve having an embedded structure used was GZ-2, the Pt load was 0.50 wt% of the carrier, and the catalyst of the present application obtained was designated as E-2.

### Example 3

80 g of sodium hydroxide, 184 g of sodium silicate, 65.6 g of sodium metaaluminate, 4.96 g of dimethyloctadecyl [3-(trimethoxysilyl)propyl] ammonium chloride (TPOAC) and 540 g of water were uniformly mixed and charged into a crystallization kettle, 42 g of ZSM-22 molecular sieve was added into the crystallization kettle and stirred, the reaction kettle was sealed, and the reaction mixture was heated to 120 °C for hydrothermal crystallization for 5 hours. After the crystallization was completed, the crystallization product obtained was washed, dried at 120 °C for 5 hours, and calcined at 500 °C for 4 hours to obtain a first molecular sieve Z3 having an embedded structure. Then, the molecular sieve Z3 was subjected to ion exchange in 1.2 mol/L CaCl₂ solution at 70 °C for 6 h, then to suction filtration and washing, then dried at 100 °C for 4 h, and then calcined at 450 °C for 3h to obtain a second molecular sieve GZ-3 having an embedded structure, of which the specific surface area was 573 m²/g, the pore volume was 0.19 mL/g, and the mass fraction of the molecular sieve having a TON structure was 47%.

A catalyst E-3 of the present application was prepared as described in Example 1, except that the molecular sieve having an embedded structure used was GZ-3, the Pt load was 0.31 wt% of the carrier, and the catalyst of the present application obtained was designated as E-3.

### Example 4

200 g of sodium hydroxide, 208g of ethyl orthosilicate, 98.4 g of sodium metaaluminate, 39.68 g of dimethyloctadecyl [3-(trimethoxysilyl)propyl] ammonium chloride (TPOAC) and 3600 g of water were uniformly mixed and charged into a crystallization kettle, 420 g of ZSM-22 molecular sieve was added and stirred, the reaction kettle was sealed, and the reaction mixture was heated to 100 °C for hydrothermal crystallization for 8 hours. After the crystallization was completed, the crystallization product obtained was washed, dried at 100 °C for 8 h, and calcined at 500 °C for 3h to obtain a first molecular sieve Z4 having an embedded structure. Then, the molecular sieve Z4 was subjected to ion exchange in 2.1 mol/L CaCl₂ solution at 100 °C for 2 h, then to suction filtration and washing, then dried at 100 °C for 3h, and then calcined at 500 °C for 3h to obtain a second molecular sieve GZ-4 having an embedded structure, of which the specific surface area was 387 m²/g, the pore volume was 0.33 mL/g, and the mass fraction of the molecular sieve having a TON structure was 89%.

A catalyst E-4 of the present application was prepared as described in Example 1, except that the molecular sieve having an embedded structure used was GZ-4, the Pt load was 0.47 wt% of the carrier, and the catalyst of the present application obtained was designated as E-4.

### Example 5

40 g of sodium hydroxide, 208g of ethyl orthosilicate, 285.6 g of aluminum isopropoxide, 5.75 g of polyethylene oxide triblock copolymer (P123) and 1440 g of water were uniformly mixed and charged into a crystallization kettle, 60 g of KZ-2 molecular sieve was added and stirred, the reaction kettle was sealed, and the reaction mixture was heated to 90 °C for hydrothermal crystallization for 10 hours. After the crystallization was completed, the crystallization product obtained was washed, dried at 110 °C for 6 h, and calcined at 450 °C for 8 h to obtain a first molecular sieve Z5 having an embedded structure. Then, the molecular sieve Z5 was subjected to ion exchange in 1.0 mol/L CaCl₂ solution at 60 °C for 8 h, then to suction filtration and washing, then dried at 100 °C for 4 h, and then calcined at 300 °C for 8 h to obtain a second molecular sieve GZ-5 having an embedded structure, of which the specific surface area was 531 m²/g, the pore volume was 0.21 mL/g, and the mass fraction of the molecular sieve having a TON structure was 53%.

A catalyst E-5 of the present application was prepared as described in Example 1, except that the molecular sieve having an embedded structure used was GZ-5, the Pt load was 0.33 wt% of the carrier, and the catalyst of the present application obtained was designated as E-5.

### Example 6

64 g of sodium hydroxide, 208g of ethyl orthosilicate, 98.4 g of sodium metaaluminate, 24.8 g of dimethyloctadecyl [3-(trimethoxysilyl)propyl] ammonium chloride (TPOAC) and 2700 g of water were uniformly mixed and charged into a crystallization kettle, then 240 g of ZSM-22 molecular sieve was added and stirred, the reaction kettle was sealed, and the reaction mixture was heated to 100 °C for hydrothermal crystallization for 7 hours. After the crystallization was completed, the crystallization product obtained was washed, dried at 100 °C for 6 hours, and calcined at 450 °C for 8 hours to obtain a first molecular sieve Z6 having an embedded structure. Then, the molecular sieve Z6 was subjected to ion exchange in 1.5 mol/L CaCl₂ solution at 80 °C for 4 h, then to suction filtration and washing, then dried at 100 °C for 5 h, and then calcined at 350 °C for 6 h to obtain a second molecular sieve GZ-6 having an embedded structure, of which the specific surface area was 498 m²/g, the pore volume was 0.23 mL/g, and the mass fraction of the molecular sieve having a TON structure was 82%.

A catalyst E-6 of the present application was prepared as described in Example 1, except that the molecular sieve having an embedded structure used was GZ-6, the Pt load was 0.48 wt% of the carrier, and the catalyst of the present application obtained was designated as E-6.

### Comparative Example 1

134 g of ZSM-22 molecular sieve (with a silica to alumina ratio of 90), 200g of 5A molecular sieve, 150 g (calculated on the basis of alumina) of aluminum hydroxide (SB available from Condean Company, Germany) and 30 g of sesbania powder were uniformly mixed, then water and concentrated nitric acid (with mass concentration of 66.5 wt%) were added, the mixture was fully kneaded to form a paste-like plastic substance. The plastic substance was extruded on a banded extruder to obtain cylindrical strips with a diameter of 1.5mm, and the cylindrical strips were dried for 12 hours at 100 °C, and then calcined for 4 hours at 550 °C in air atmosphere to obtain a catalyst carrier ES-7 of the present application.

300 g of the carrier ES-7 was impregnated with the noble metal Pt via saturated impregnation, and then the resultant was dried for 7 hours at 110 °C, and calcined for 3 hours at 500 °C in air atmosphere to obtain a catalyst of the present application comprising 0.49 wt% of Pt, designated as C-1.

### Comparative Example 2

40 g of sodium hydroxide, 208g of ethyl orthosilicate, 285.6 g of aluminum isopropoxide, 5.75 g of polyethylene oxide triblock copolymer (P123) and 1440 g of water were uniformly mixed and charged into a crystallization kettle, then 60 g Hβ molecular sieve was added and stirred, the reaction kettle was sealed, and the reaction mixture was heated to 90 °C for hydrothermal crystallization for 10 hours. After the crystallization was completed, the crystallization product obtained was washed, dried at 110 °C for 6 h, and calcined at 450 °C for 8 h to obtain a first molecular sieve Z7 having an embedded structure. Then the molecular sieve Z7 was subjected to ion exchange in 1.8mol/L CaCl₂ solution at 90 °C for 4 hours, then to suction filtration and washing, then dried at 100 °C for 4 hours, and calcined at 400 °C for 3 hours to obtain a second molecular sieve GZ-7 having an embedded structure.

A comparative catalyst C-2 of the present application was prepared as described in Example 1, except that the molecular sieve having an embedded structure used was GZ-7, the Pt load was 0.30 wt% of the carrier, and the catalyst of the present application obtained was designated as C-2.

**Table 1 Major physicochemical properties of catalysts obtained in the examples/comparative examples**

| Catalyst | Molecular sieve | Pt/wt% | S/(m²/g) | V/(mL/g) |
|---|---|---|---|---|
| E-1 | GZ-1 | 0.28 | 382 | 0.30 |
| E-2 | GZ-2 | 0.50 | 363 | 0.33 |
| E-3 | GZ-3 | 0.31 | 393 | 0.31 |
| E-4 | GZ-4 | 0.47 | 325 | 0.35 |
| E-5 | GZ-5 | 0.33 | 371 | 0.32 |
| E-6 | GZ-6 | 0.48 | 416 | 0.29 |
| C-1 | ZSM-22/5A | 0.49 | 374 | 0.27 |
| C-2 | GZ-7 | 0.30 | 615 | 0.41 |

Five types of feedstock oils were used in the examples and comparative examples of the method of the present application, namely hydrocracking UCO, hydrotreated deasphalted oil, hydrotreated wax oil, hydrotreated slack wax and hydrotreated foots oil, and the properties of the feedstock are shown in Table 3.

**Table 3 Properties of the feedstock oils**

| Feedstock oil | Hydrocracking UCO | Hdrotreated deasphalted oil | Hydrotreated wax oil | Hydrotreated slack wax | Hydrotreated foots oil |
|---|---|---|---|---|---|
| Density, kg/m³ | 835 | 895 | 857 | 822 | 842 |
| Distillation range, °C | 375-530 | 350-750 | 386-515 | 380-480 | 370-510 |
| Viscosity (100 °C), mm2/s | 4.02 | 9.87 | 5.63 | 3.85 | 4.51 |
| Pour point, °C | 36 | 45 | 41 | 49 | 38 |
| Sulfur, µg/g | 12.5 | 20.0 | 9.6 | 5.8 | 6.5 |
| Nitrogen, µg/g | <1.0 | 2.5 | 1.8 | 1.0 | 1.2 |
| Refractive index (20 °C) | 1.4680 | 1.5984 | 1.4921 | 1.4342 | 1.4511 |

| Mass spectrum composition, % | | | | | |
|---|---|---|---|---|---|
| Paraffins | 57.7 | 38.6 | 48.3 | 77.25 | 55.8 |
| Naphthenes | 39.0 | 48.9 | 41.93 | 19.85 | 40.6 |
| Monocyclic/bicyclic/tricyclic | 16.5/14.6/5.0 | 22.4/20.2/4.0 | 17.9/20.43/3.3 | 9.75/7.31/2.55 | 17.7/16.2/5.3 |
| Tetracyclic/pentacyclic/hexacyclic | 2.9/-/- | 1.6/0.5/0.2 | 0.3/-/- | 0.24/-/- | 1.4/-/- |
| Aromatics | 3.3 | 12.5 | 9.77 | 2.9 | 3.6 |
| Monocyclic/bicyclic | 2.5/0.5 | 7.6/3.5 | 6.11/2.66 | 2.1/0.5 | 4.2/1.5 |
| Tricyclic/tetracyclic/pentacyclic | 0.2/0.1/- | 0.8/0.4/0.1 | 0.8/0.2/- | 0.2/0.1/- | 0.2/0.1/- |
| Not identified | - | 0.1 | - | - | - |

### Examples 8 to 15

Examples 8 to 15 were carried out using the hydrocracking UCO shown in Table 3 as feedstock in accordance with the process flow shown in Fig. 1, with catalysts E-2, E-6, E-4, E-5, E-3, E-1, E-2 and E-2 being respectively packed in the adsorption reaction zone. The operation conditions and results are shown in Table 4.

**Table 4 Operation conditions and results of Examples 8-15**

| Example No. | Example 8 | Example 9 | Example 10 | Example 11 | Example 12 | Example 13 | Example 14 | Example 15 | Comp. Ex. 1 | Comp. Ex. 2 |
|---|---|---|---|---|---|---|---|---|---|---|
| Catalyst | E-2 | E-6 | E-4 | E-5 | E-3 | E-1 | E-2 | E-2 | C-1 | C-2 |
| Feedstock oil | Hydrocr acking UCO | Hydrocr acking UCO | Hydrocr acking UCO | Hydrocr acking UCO | Hydroc racking UCO | Hydrocra cking UCO | Hydrocra cking UCO | Hydrocra cking UCO | Hydroc racking UCO | Hydrocr acking UCO |

| Adsorption reaction conditions of the first reaction zone | | | | | | | | | | |
|---|---|---|---|---|---|---|---|---|---|---|
| Adsorption temperature /°C | 130 | 130 | 130 | 130 | 130 | 130 | 130 | 130 | 130 | 130 |
| Adsorption pressure /MPa | 0.09 | 0.09 | 0.09 | 0.09 | 0.09 | 0.09 | 0.09 | 0.09 | 0.09 | 0.09 |
| Volume space velocity /h⁻¹ | 1.0 | 1.0 | 1.0 | 1.0 | 1.0 | 1.0 | 1.0 | 1.0 | 1.0 | 1.0 |
| Refractive index of the first stream (20 °C) | 1.4900 | 1.4900 | 1.4900 | 1.4900 | 1.4900 | 1.4900 | 1.4900 | 1.4900 | 1.4900 | 1.4900 |
| Increase in refractive index of the first stream over that of the feedstock /% | 1.5 | 1.5 | 1.5 | 1.5 | 1.5 | 1.5 | 0.3 | 3.0 | 1.5 | 1.5 |

| Hydroisomerization conditions of the first reaction zone | | | | | | | | | | |
|---|---|---|---|---|---|---|---|---|---|---|
| Reaction temperature /°C | 325 | 325 | 325 | 325 | 325 | 325 | 325 | 325 | 325 | 325 |
| Reaction pressure /MPa | 10.0 | 10.0 | 10.0 | 10.0 | 10.0 | 10.0 | 10.0 | 10.0 | 10.0 | 10.0 |
| Volume ratio of hydrogen to oil | 300 | 300 | 300 | 300 | 300 | 300 | 300 | 300 | 300 | 300 |
| Reaction time /h | 1.2 | 1.2 | 1.2 | 1.2 | 1.2 | 1.2 | 1.2 | 1.2 | 1.2 | 1.2 |
| Conditions of vacuum distillation | 11 | 11 | 11 | 11 | 11 | 11 | 11 | 11 | 11 | 11 |
| Top pressure of the distillation column /mmHg | | | | | | | | | | |
| Bottom temperature of the distillation column /°C | 320 | 320 | 320 | 320 | 320 | 320 | 320 | 320 | 320 | 320 |

| Base oil Properties | | | | | | | | | | |
|---|---|---|---|---|---|---|---|---|---|---|
| Light lubricant base oil | | | | | | | | | | |
| Pour point /°C | -30 | -24 | -23 | -20 | -16 | -13 | -30 | -26 | -9 | -6 |
| Viscosity (100 °C), mm/s² | 2.100 | 2.108 | 2.093 | 2.089 | 2.120 | 2.097 | 2.106 | 2.131 | 2.079 | 2.108 |
| Viscosity index | 100 | 99 | 98 | 98 | 99 | 97 | 98 | 104 | 93 | 88 |

| Medium lubricant base oil | | | | | | | | | | |
|---|---|---|---|---|---|---|---|---|---|---|
| Pour point /°C | -27 | -22 | -20 | -18 | -14 | -10 | -27 | -22 | -7 | -3 |
| Viscosity (100 °C), mm/s² | 4.020 | 4.035 | 4.010 | 4.000 | 4.108 | 4.050 | 4.065 | 4.200 | 4.061 | 4.036 |
| Viscosity index | 116 | 113 | 113 | 113 | 110 | 112 | 112 | 122 | 106 | 101 |

| Heavy lubricant base stock | | | | | | | | | | |
|---|---|---|---|---|---|---|---|---|---|---|
| Pour point /°C | -15 | -11 | -10 | -9 | -7 | -4 | -14 | -11 | -2 | 1 |
| Viscosity (100 °C), mm/s² | 6.011 | 6.020 | 6.110 | 6.000 | 6.100 | 6.036 | 6.155 | 6.201 | 6.160 | 6.032 |
| Viscosity index | 125 | 121 | 121 | 120 | 120 | 119 | 122 | 129 | 111 | 109 |

### Examples 16 to 19

Examples 16 to 19 were carried out using the hydrotreated deasphalted oil, hydrotreated wax oil, hydrotreated slack wax, and hydtreated foots oil shown in Table 3 as feedstock in accordance with the process flow shown in Fig. 2, with Catalyst E-2 being packed in both the first and second adsorption reaction zones. The operation conditions and results are shown in Table 5.

**Table 5 Operation conditions and results of Examples 16-19**

| Example No. | | Example 16 | Example 17 | Example 18 | Example 19 |
|---|---|---|---|---|---|
| Catalyst | | E-2 | E-2 | E-2 | E-2 |
| Feedstock oil | | Hdrotreated deasphalted oil | Hdrotreated wax oil | Hdrotreated slack wax | Hdrotreated foots oil |

| Adsorption reaction conditions of the first reaction zone | | | | | |
|---|---|---|---|---|---|
| Adsorption temperature /°C | | 230 | 190 | 120 | 50 |
| Adsorption pressure /MPa | | 0.02 | 0.12 | 0.30 | 0.45 |
| Volume space velocity /h⁻¹ | | 4.6 | 2.5 | 0.08 | 1.60 |
| Refractive index of the first stream (20 °C) | | 1.6000 | 1.5040 | 1.5059 | 1.4888 |
| Increase in refractive index of the first stream over that of the feedstock /% | | 0.1 | 0.8 | 5.0 | 2.6 |

| Hydroisomerization conditions of the first reaction zone | | | | | |
|---|---|---|---|---|---|
| Reaction temperature /°C | | 400 | 250 | 350 | 280 |
| Reaction pressure /MPa | | 2.0 | 18.0 | 12.0 | 8.0 |
| Volume ratio of hydrogen to oil | | 1500 | 300 | 1000 | 600 |
| Reaction time /h | | 2 | 0.5 | 9 | 5 |

| Adsorption reaction conditions of the second reaction zone | | | | | |
|---|---|---|---|---|---|
| Adsorption temperature /°C | | 230 | 190 | 120 | 50 |
| Adsorption pressure /MPa | | 0.02 | 0.12 | 0.30 | 0.45 |
| Volume space velocity /h⁻¹ | | 4.6 | 2.5 | 0.08 | 1.60 |
| Refractive index of the first stream (20 °C) | | 1.6000 | 1.5040 | 1.5059 | 1.4888 |
| Increase in refractive index of the first stream over that of the feedstock /% | | 0.1 | 0.8 | 5.0 | 2.6 |

| Hydroisomerization conditions of the second reaction zone | | | | | |
|---|---|---|---|---|---|
| Reaction temperature /°C | | 400 | 250 | 350 | 280 |
| Reaction pressure /MPa | | 2.0 | 18.0 | 12.0 | 8.0 |
| Volume ratio of hydrogen to oil | | 1500 | 300 | 1000 | 600 |
| Reaction time /h | | 2 | 0.5 | 9 | 5 |

| Conditions of vacuum distillation | | | | | |
|---|---|---|---|---|---|
| Top pressure of the distillation column /mmHg | | 100 | 50 | 1 | 23 |
| Bottom temperature of the distillation column /°C | | 200 | 280 | 370 | 330 |

| Light lubricant base oil | | | | | |
|---|---|---|---|---|---|
| | Pour point /°C | -35 | -24 | -33 | -28 |
| | Viscosity (100 °C), mm/s² | 2.45 | 2.21 | 1.98 | 2.06 |
| | Viscosity index | 96 | 104 | 108 | 110 |

| Medium lubricant base oil | | | | | |
|---|---|---|---|---|---|
| | Pour point /°C | -31 | -20 | -30 | -25 |
| | Viscosity (100 °C), mm/s² | 8.033 | 5.510 | 3.702 | 3.920 |
| | Viscosity index | 112 | 118 | 121 | 125 |

| Heavy lubricant base stock | | | | | |
|---|---|---|---|---|---|
| | Pour point /°C | -17 | -8 | -15 | -13 |
| | Viscosity (100 °C), mm/s² | 13.71 | 11.90 | 9.10 | 10.80 |
| | Viscosity index | 121 | 127 | 130 | 132 |

### Comparative Example 1

An experiment was carried out using the hydrocracking UCO shown in Table 3 as feedstock in accordance with the process flow shown in Fig. 1, with Catalyst C-1 being packed in the adsorption reaction zone. The operation conditions and results are shown in Table 3.

### Comparative Example 2

An experiment was carried out using the hydrocracking UCO shown in Table 3 as feedstock in accordance with the process flow shown in Fig. 1, with Catalyst C-2 being packed in the adsorption reaction zone. The operation conditions and results are shown in Table 3.

## Claims

1. A molecular sieve composition, comprising a molecular sieve having an embedded structure composed of a molecular sieve having a TON structure and a 5A-type molecular sieve, preferably the molecular sieve having a TON structure is embedded onto at least a part of the surface of the 5A-type molecular sieve.

2. The molecular sieve composition according to claim 1, wherein the calcined form of the molecular sieve having an embedded structure shows an XRD pattern substantially as shown in Table I or Table II below, preferably having an XRD pattern substantially as shown in Fig. 1,
**Table I**
| **2θ** | **d-spacing (nm)** | **Relative strength** |
|---|---|---|
| 7.184 | 1.204±0.020 | W-S |
| 8.229 | 1.090±0.020 | M-VS |
| 10.133 | 0.872±0.006 | M |
| 12.473 | 0.709±0.008 | W-M |
| 12.685 | 0.694±0.006 | W-M |
| 16.140 | 0.549±0.004 | W |
| 19.338 | 0.459±0.007 | W |
| 20.275 | 0.438±0.008 | M-VS |
| 21.699 | 0.409±0.008 | W-S |
| 24.098 | 0.369±0.005 | VS |
| 24.563 | 0.362±0.004 | M-VS |
| 25.680 | 0.347±0.005 | W-M |
| 26.148 | 0.341±0.003 | W |
| 27.152 | 0.328±0.002 | W |
| 29.997 | 0.298±0.003 | W |
| 34.237 | 0.262±0.005 | W-M |
| 35.537 | 0.252±0.006 | W-M |
**Table II**
| **2θ** | **d-spacing (nm)** | **Relative strength** |
|---|---|---|
| 7.184 | 1.204±0.020 | W-S |
| 8.229 | 1.090±0.020 | M-VS |
| 10.133 | 0.872±0.006 | M |
| 12.473 | 0.709±0.008 | W-M |
| 12.685 | 0.694±0.006 | W-M |
| 14.399 | 0.615±0.006 | W |
| 16.140 | 0.549±0.004 | W |
| 17.630 | 0.503±0.006 | W |
| 19.338 | 0.459±0.007 | W |
| 20.275 | 0.438±0.008 | M-VS |
| 21.699 | 0.409±0.008 | W-S |
| 24.098 | 0.369±0.005 | VS |
| 24.563 | 0.362±0.004 | M-VS |
| 25.680 | 0.347±0.005 | W-M |
| 26.148 | 0.341±0.003 | W |
| 26.675 | 0.334±0.004 | W |
| 27.152 | 0.328±0.002 | W |
| 29.997 | 0.298±0.003 | W |
| 30.725 | 0.290±0.004 | W-M |
| 32.624 | 0.274±0.005 | W |
| 34.237 | 0.262±0.005 | W-M |
| 35.537 | 0.252±0.006 | W-M |
| 36.835 | 0.244±0.004 | W |
| 38.038 | 0.236±0.004 | W |
wherein, the intensity value of the strongest diffraction peak in the XRD pattern is set as 100, and W represents weak, namely a relative intensity from more than 0 to less than or equal to 20; M represents medium, namely a relative strength from more than 20 to less than or equal to 40; S represents strong, namely a relative strength from more than 40 to less than or equal to 60; VS represents very strong, that is a relative intensity from more than 60 to less than or equal to 100.

3. The molecular sieve composition according to claim 1, wherein the molecular sieve having an embedded structure has a specific surface area of from 300 m²/g to 600m ²/g and a pore volume of from 0.15 cm³/g to 0.40 cm³/g, or the molecular sieve composition has a specific surface area of from 200 m²/g to 550 m²/g and a pore volume of from 0.25 cm³/g to 0.60 cm³/g.

4. The molecular sieve composition according to claim 1, wherein in the molecular sieve having an embedded structure, the weight ratio of the 5A-type molecular sieve to the molecular sieve having a TON structure is 1 : 80 to 3 : 1, preferably 1 : 30 to 1 : 1.

5. The molecular sieve composition according to claim 1, wherein the molecular sieve having a TON structure is present in an amount of from 10wt% to 80wt%, preferably from 20wt% to 60wt%, the 5A-type molecular sieve is present in an amount of from 1wt% to 50wt%, preferably from 2wt% to 20wt%, relative to the total weight of the molecular sieve composition as 100wt%, or the molecular sieve having an embedded structure is present in an amount of from 10wt% to 90wt%, preferably from 20wt% to 70wt%, relative to the total weight of the molecular sieve composition as 100wt%.

6. The molecular sieve composition according to claim 1, further comprising an inorganic refractory oxide and an active metal component, wherein the active metal component is present in an amount of 0.05wt% to 5.0wt%, preferably 0.1wt% to 1.0wt%, calculated on the basis of the metal element, relative to the total weight of the molecular sieve composition as 100wt%.

7. The molecular sieve composition according to claim 6, wherein the inorganic refractory oxide is one or more selected from the group consisting of alumina, titania, boria, silica, zirconia and magnesia, preferably alumina, and the active metal component is at least one selected from the Group VIII noble metals of the periodic table, preferably at least one selected from Pt and Pd, especially Pt.

8. The molecular sieve composition according to claim 1, wherein the molecular sieve having a TON structure is one or more selected from the group consisting of ZSM-22, Theta-1, ISI-1, KZ-2 and NU-10, preferably ZSM-22, and the 5A-type molecular sieve is selected from 5A molecular sieves.

9. The molecular sieve composition according to claim 1, having dual functions of adsorption and hydroisomerization.

10. A method for preparing a molecular sieve having an embedded structure, comprising the steps of:
(1) contacting a silicon source, an aluminum source, and an alkali source in the presence of a templating agent, a molecular sieve having a TON structure, and water to obtain a gel mixture, and
(2) carrying out hydrothermal crystallization on the gel mixture, and then washing, drying and calcining to obtain a first molecular sieve having an embedded structure.

11. The method according to claim 10, further comprising the steps of:
(3) carrying out calcium exchange on the first molecular sieve having an embedded structure, and then washing, drying and calcining to obtain a second molecular sieve having an embedded structure.

12. The method according to claim 10, wherein in step (1), the silicon source is at least one selected from the group consisting of water glass, sodium silicate, methyl orthosilicate, ethyl orthosilicate, propyl orthosilicate and butyl orthosilicate, the alkali source is at least one of alkali metal hydroxides, preferably sodium hydroxide, and the aluminum source is at least one selected from the group consisting of sodium metaaluminate, aluminum isopropoxide, aluminum sulfate, aluminum hydroxide, aluminum oxide and pseudo-boehmite, the template agent is at least one selected from polyethylene oxide triblock copolymer (P123) and dimethyloctadecyl[3-(trimethoxysilyl)propyl] ammonium chloride (TPOAC), the molecular sieve having a TON structure is one or more selected from the group consisting of ZSM-22, Theta-1, ISI-1, KZ-2 and NU-10, preferably ZSM-22.

13. The method as claimed in claim 10, wherein in step (1), the operating conditions of the contacting include: under stirring, a temperature of 15-30 °C, and a time period of 1-4 h; the molar ratio of the alkali source (calculated as oxide), the silicon source (calculated as SiO₂), the aluminum source (calculated by Al₂O₃), the template agent and water is (0.5-2.5) : 1 : (0.4-0.7): (0.001-0.08) : (30-200), wherein the amount of the molecular sieve having a TON structure is 0.3-8 times (preferably 1-7 times) of the amount of the silicon source.

14. The method according to claim 10, wherein in step (2), the hydrothermal crystallization temperature is 60 °C to 120 °C, the crystallization time is 2h to 16h, the drying temperature is 80 °C to 200 °C, the drying time is 2h to 24h, the calcining temperature is 400 °C to 600 °C, and the calcining time is 2h to 12 h.

15. The method according to claim 10, wherein in step (3), the operating conditions for the calcium exchange include: an exchange temperature of 60-100 °C, an exchange time of 1-12 h, and a calcium ion concentration in the calcium exchange solution of 0.1-2.5 mol/L; a drying temperature of 80-150 °C, a drying time of 2-12 h, a calcining temperature of 300-500 °C, and a calcining time of 2-8 h.

16. A method for producing a lubricant base oil, comprising the steps of:
(1) contacting a feedstock comprising a long-side-chain hydrocarbon with the molecular sieve composition according to claim 1 (for example in the presence or absence of hydrogen) to obtain an enriched molecular sieve composition and a raffinate having a reduced content of the long-side-chain hydrocarbon, wherein the long-side-chain hydrocarbon is one or more selected from the group consisting of long-side-chain isoparaffins, long-side-chain monocyclic naphthenes, and long-side-chain monocyclic aromatics,
(2) subjecting the enriched molecular sieve composition to a hydroisomerization in the presence of hydrogen to obtain an isomerization product mixture and a depleted molecular sieve composition, and
(3) separating the isomerization product mixture to obtain the lubricant base oil.

17. The method according to claim 16, wherein the feedstock is contacted with the molecular sieve composition repeatedly a plurality of times until the refractive index of the raffinate is from 0.1% to 5% (preferably from 0.5% to 2.5%) higher than the refractive index of the feedstock.

18. The method according to claim 16, wherein the feedstock is one or more selected from the group consisting of hydrocracking UCO, hydrotreated wax oil, hydrotreated bright stock, hydrotreated slack wax and hydrotreated foots oil.

19. The method according to claim 16, wherein in step (1), the operating conditions of the contacting include: a temperature of 40-250 °C, preferably 60-200 °C, a pressure of 0.01-0.5 MPa, preferably 0.08-0.1MPa, and a volume space velocity of 0.05-5.0 h⁻¹ , preferably 0.1-2.0 h⁻¹ .

20. The method according to claim 16, wherein in step (2), the operating conditions of the hydroisomerization include: the presence of hydrogen, a reaction temperature of 200-420 °C, preferably 270-380 °C, a hydrogen partial pressure of 1.0-20.0MPa, preferably 3.0-15.0MPa, a reaction time of 0.1-10 h, preferably 0.3-2 h, and a volume ratio of hydrogen to oil of 100 : 1 to 1500 : 1, preferably 100 : 1 to 400 : 1.

21. The method according to claim 16, wherein in step (3), the separation comprises the steps of:
(3-1) subjecting the isomerization product mixture to gas-liquid separation to obtain a gas stream and a liquid stream, optionally recycling at least a part of the gas stream to step (1) as at least a part of the feedstock, and
(3-2) subjecting the liquid stream to fractionation to obtain the lubricant base oil, preferably to obtain a light lubricant base oil, a medium lubricant base oil and a heavy lubricant base stock, and optionally recycling at least a part of the heavy lubricant base stock to step (1) as at least a part of the feedstock.

22. The method according to claim 16, wherein in step (3-1), the gas-liquid separation is performed by vacuum distillation under conditions including: a pressure at the top of the distillation column of 1-100 mm Hg, preferably 2-20 mm Hg, and a temperature at the bottom of the distillation column of 200-370 °C, preferably 290-350 °C, or in step (3-2), the fractionation is performed by vacuum distillation under conditions including: a pressure at the top of the distillation column of 1-100 mm Hg, preferably 2-20 mm Hg, and a temperature at the bottom of the distillation column of 200-370 °C, preferably 290-350 °C.

23. The method according to claim 16, wherein the step (1) and the step (2) are carried out in the same reactor.

24. A system for producing lubricant base oils, comprising:
one or more reaction units packed with the molecular sieve composition according to claim 1 and configured to contact a feedstock comprising a long-side-chain hydrocarbon with the molecular sieve composition according to claim 1 to obtain an enriched molecular sieve composition and a raffinate having a reduced content of the long-side-chain hydrocarbon, and then treating the enriched molecular sieve composition under hydroisomerization conditions to obtain an isomerization product mixture and a depleted molecular sieve composition,
a gas-liquid separation unit configured to carry out a gas-liquid separation on the isomerization product mixture to obtain a gas stream and a liquid stream, and
a fractionation column configured to fractionate the liquid stream to obtain the lubricant base oil.
